# EUROPÄISCHE PATENTSCHRIFT

(11) **EP 2 572 686 B1**
(45) Veröffentlichungstag und Bekanntmachung des Hinweises auf die Patenterteilung: **30.07.2014**
(21) Anmeldenummer: 11182545.1
(22) Anmeldetag: 23.09.2011
(51) Int. Cl.: A61F 5/56

(54) **Vorgefertigte Zahnschutzschiene**
Pre-fabricated teeth protection rail
Rail de protection dentaire préfabriqué

(43) Veröffentlichungstag der Anmeldung: 27.03.2013
(73) Patentinhaber: 2G medical GmbH, 3074 Muri b. Bern (CH)
(72) Erfinder: Grohmann, Philipp, 8049 Zürich (CH); Gigandet, Michel, 3076 Worb (CH)
(74) Vertreter: AMMANN PATENTANWÄLTE AG BERN

(56) Entgegenhaltungen:
- WO-A1-2007/113465
- WO-A2-03/051280
- US-A1- 2010 009 311
- US-A1- 2011 201 970

## Beschreibung

Die Erfindung betrifft eine vorgefertigte Zahnschutzschiene mit einer durch eine Vorderwand und eine Rückwand begrenzten Mulde zur Aufnahme von Schneidezähnen einer Person, und einer zur Anlage an den in der Mulde aufgenommenen Schneidezähnen gegenüberliegenden Schneidezähnen bestimmte Kontaktfläche, wobei die Kontaktfläche in einer Richtung rechtwinklig zum Verlauf der Mulde zumindest in der Längsmitte der Mulde deren Breite auf beiden Seiten überragt.

Zahnschutzschienen verhindern den direkten Kontakt von Zähnen des Oberkiefers mit Zähnen des Unterkiefers. Sie werden beispielsweise bei Patienten eingesetzt, die unter Bruxismus leiden, einem unbewussten, meist nächtlichen Zähneknirschen, bei dem die Zähne aufeinander gepresst und durch Bewegungen des Unterkiefers aneinander gerieben werden. Ausserdem werden solche Sperrvorrichten auch bei Patienten eingesetzt, die unter Spannungskopfschmerzen leiden und bei denen der Verdacht besteht, dass sie - insbesondere im Schlaf - die Zähne aufeinander pressen und dabei die Kiefermuskulatur stark und längerdauernd isometrisch anspannen.

Bekannte Zahnschutzschienen, wie sie beispielsweise in den Dokumenten WO9629950A1, WO2003051280A2 und WO03051244A1 beschrieben sind, bestehen aus einem vorgefertigten Teil, meistens aus spritzgegossenem Kunststoff, das durch den Zahnarzt einmalig mittels einer aushärtbaren Masse an die oberen oder unteren Schneidezähne exakt angepasst wird, so dass die Zahnschutzschiene für die tägliche Verwendung vom Patienten auf die Schneidezähnen aufgesetzt und nach Gebrauch wieder abgenommen werden kann.

Die bekannten Zahnschutzschienen weisen eine Kontaktfläche zur Anlage an den Schneidezähnen auf, die den Schneidezähnen gegenüberliegen, an denen die Zahnschutzschiene angebracht ist. Somit verhindern diese Zahnschutzschienen den direkten Kontakt nicht nur der Schneidezähne, sondern auch der benachbarten Backenzähne. Die Kontaktfläche ist dabei im Wesentlichen rechtwinklig zur Mulde orientiert und ragt vorne und hinten über den Bereich der Mulde hinaus. Damit wird gewährleistet, dass auch bei einem starken Vorbiss oder Überbiss die der Zahnschutzschiene gegenüberliegenden Schneidezähne stets die Kontaktfläche berühren und sich beim Schliessen des Kiefers nicht innen oder aussen an der Zahnschutzschiene vorbei bewegen können, was zu einem unerwünschten Kontakt der Backenzähne des Oberkiefers mit Backenzähnen des Unterkiefers führen könnte.

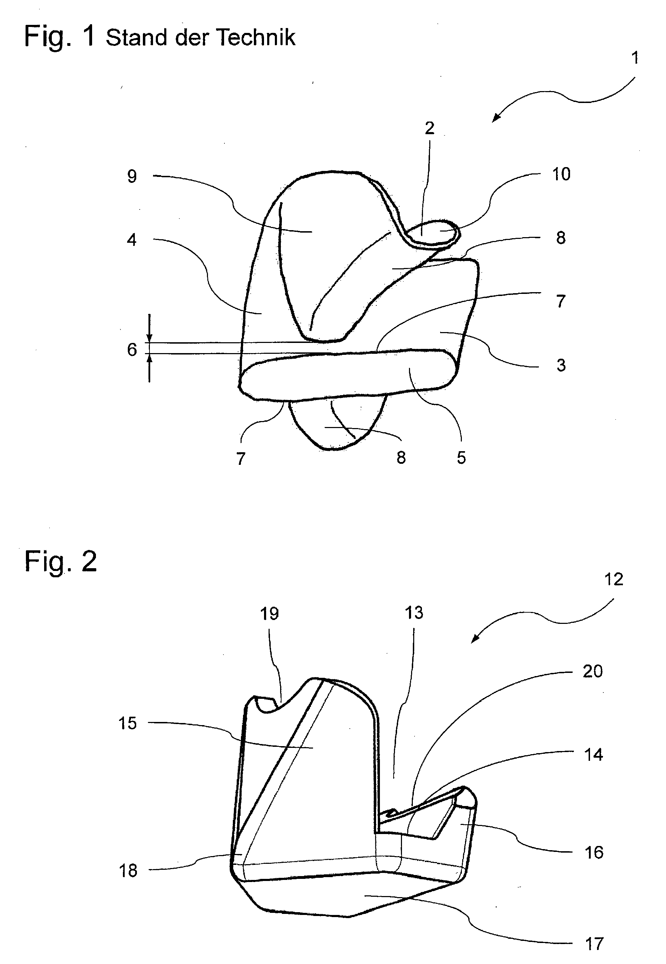

Bei den bekannten Zahnschutzschienen ist die beschriebene Kontaktfläche zumindest in den Bereichen innerhalb der Mulde im Gaumenbereich und ausserhalb der Mulde im Lippenbereich durch seitliche Kanten begrenzt. Bei einer bekannten Zahnschutzschiene, die in Figur 1 dargestellt ist und weiter unten genauer beschrieben wird, ist die Kontaktfläche gegenüber der Aussenwand der Mulde abgesetzt, so dass sich diese seitlichen Kanten sogar von aussen nach innen über die gesamte Länge der Kontaktfläche erstrecken.

Bei den meisten Menschen sind nicht alle nebeneinander liegenden Schneidezähne genau gleich lang. Daraus ergibt sich ein gemeinsamer Nachteil der erwähnten bekannten Zahnschutzschienen, der darin besteht, dass mindestens ein Schneidezahn bei Lateralbewegungen des Unterkiefers an einer der genannten Kanten der Zahnschutzschiene anstehen und dadurch zu einem unerwünschten Aufbau von Spannung der Kiefermuskulatur und einer ebenso unerwünschten mechanischen Belastung des betreffenden Schneidezahnes und der Zahnschutzschiene führen kann. Die Belastung des Schneidezahns kann zu Schmerzen im Wurzelbereich führen und die Belastung der Zahnschutzschiene zu einer unerwünschten Lockerung im unterfütterten Bereich der Mulde.

Ausgehend von diesem Stand der Technik liegt der Erfindung die Aufgabe zu Grunde, eine Zahnschutzschiene der eingangs genannten Art vorzuschlagen, welche den genannten Nachteil des Standes der Technik nicht aufweist und insbesondere ungehinderte Lateralbewegungen des Unterkiefers zulässt.

Diese Aufgaben werden erfindungsgemäss dadurch gelöst, dass sich die Kontaktfläche zumindest im Bereich der Mulde über deren ganze Länge erstreckt. Als Länge der Mulde soll im vorliegenden Zusammenhang die Strecke von einem offenen Ende zum gegenüberliegenden offenen Ende der Mulde verstanden werden.

Diese erfindungsgemässe Lösung hat insbesondere den Vorteil, dass durch die verbreiterte Kontaktfläche relativ grosse Lateralbewegungen der Kiefer ausgeführt werden können, ohne dass durch Anstehen eines gegenüberliegenden Zahnes an einer Kante der Kontaktfläche unerwünschte Lateralkräfte aufgebaut werden.

Nach einer Ausführungsart ist die Kontaktfläche eine ebene Fläche. Dadurch können die gegenüberliegenden Zähne in einem weiten Bereich auf dieser Fläche gleiten, ohne dass die Kieferöffnung verändert wird und ohne dass unerwünschte Kräfte, insbesondere Lateralkräfte aufgebaut werden.

Nach einer weiteren Ausführungsart nimmt in der Längsmitte der Mulde die Frontwand von ihrem freien Rand in Richtung auf die Kontaktfläche in ihrer Dicke zu und die Dicke der Frontwand nimmt im Bereich der Kontaktfläche von der Mitte aus zu beiden Enden der Mulde hin stetig ab. Durch diese Massnahme wird die Erstreckung der Kontaktfläche nach vorne ohne Irritation der Lippen einer die Zahnschutzschiene tragenden Person gewährleistet.

Eine weitere Ausführungsart sieht vor, dass die Rückwand von ihrem freien Rand in Richtung auf die Kontaktfläche in ihrer Dicke zunimmt. Durch diese Massnahme wird die Erstreckung der Kontaktfläche nach hinten ohne Irritation des Rachens und der Zunge einer die Zahnschutzschiene tragenden Person gewährleistet.

Gemäss einer anderen Ausführungsart ist die der Mulde abgewandte Seite der Rückwand eine ebene Fläche. Somit verläuft der Übergang zwischen der Rückwand und der Kontaktfläche entlang einer Sehne des Zahnbogens. Es hat sich gezeigt, dass eine solche Gestaltung den Rachen und die Zunge einer die Zahnschutzschiene tragenden Person wesentlich weniger irritiert, als etwa der aus dem Dokument WO03051244A1 bekannte Vorsprung.

Wenn nach einer weiteren Ausführungsart der freie Rand der Frontwand in deren Mitte eine Ausnehmung aufweist, wird verhindert, dass beim Tragen der Zahnschutzschiene an den oberen Schneidezähnen das obere Lippenbändchen beziehungsweise beim Tragen der Zahnschutzschiene an den unteren Schneidezähnen das untere Lippenbändchen durch die freie Kante der Frontwand irritiert und/oder gereizt wird.

Wenn nach einer weiteren Ausführungsart der freie Rand der Rückwand in deren Mitte eine Ausnehmung aufweist, wird verhindert, dass beim Tragen der Zahnschutzschiene an den oberen Schneidezähnen die Erhebung am vorderen Gaumenende beziehungsweise beim Tragen der Zahnschutzschiene an den unteren Schneidezähnen das Zungenbändchen durch die freie Kante der Rückwand irritiert und/oder gereizt wird.

Nach einer anderen Ausführungsart beträgt die Länge der Mulde gemessen an der Bodenfläche der Mulde entlang der Vorderwand zwischen 16 mm und 35 mm. Dadurch ist die Kontaktfläche entlang der Inzisalkante deutlich länger als bei den aus dem Stand der Technik bekannten Zahnschutzschienen.

Schliesslich ist nach einer weiteren Ausführungsart vorgesehen, dass die Distanz von der Bodenfläche der Mulde bis zur Kontaktfläche zwischen 0,5 mm und 3 mm beträgt. Dadurch sind die Kiefer einer die Zahnschutzschiene tragenden Person möglichst wenig gespreizt, was nicht nur angenehmer ist als eine starke Spreizung, sondern auch das Risiko der Bildung eines offenen Bisses reduziert.

Ein Ausführungsbeispiel der Erfindung wird nachstehend unter Bezugnahme auf die angefügten Zeichnungen beispielsweise näher beschrieben. Es zeigt
- Figur 1: eine aus dem Stand der Technik bekannte Zahnschutzschiene in einer perspektivischen Ansicht,
- Figur 2: eine perspektivische Ansicht eines Ausführungsbeispiels der erfindungsgemässen Zahnschutzschiene,
- Figur 3: eine entlang der Linie III - III in Figur 5 geschnittene Ansicht der Zahnschutzschiene von Figur 2,
- Figur 4: eine Ansicht der Zahnschutzschiene von Figur 2 von vorne,
- Figur 5: eine Ansicht der Zahnschutzschiene von Figur 2 von oben,
- Figur 6: eine Ansicht der Zahnschutzschiene von Figur 2 von hinten und
- Figur 7: eine Ansicht der Zahnschutzschiene von Figur 2 von unten.

In Figur 1 ist als Beispiel für den bekannten Stand der Technik eine im Handel erhältliche Zahnschutzschiene 1 in einer perspektivischen Ansicht von seitlich unten dargestellt. In der vorliegenden Beschreibung wird angenommen, dass die Zahnschutzschiene an den oberen Schneidezähnen einer Person angebracht wird. Dem entsprechend werden die Bezeichnungen "oben" und "unten" entsprechend der Darstellung in den Figuren 1 und 2 verwendet. Ausserdem wird in der gesamten nachfolgenden Beschreibung die Bezeichnung "vorne" für die den Lippen einer die Zahnschutzschiene 1 tragenden Person zugewandten Bereiche verwendet und die Bezeichnung "hinten" für die dem Rachen zugewandten Bereiche. Die Zahnschutzschiene 1 weist eine bogenförmig verlaufende Mulde 2 auf, die zur Aufnahme von Schneidezähnen einer Person vorgesehen ist und durch eine Vorderwand 9 und eine Rückwand 10 begrenzt ist. Die innere, entlang der Vorderwand 9 gemessene Länge der Mulde 2 beträgt im beschriebenen Beispiel 32 mm, so dass sie etwa vier Schneidezähne aufnehmen kann. Zur genauen Anpassung wird die Mulde 2 vom Zahnarzt mit einer aushärtbaren plastischen Masse gefüllt, über die Schneidezähne gedrückt und fixiert, bis die Masse ausgehärtet ist. Eine Kontaktfläche 5 für die gegenüberliegenden Schneidezähne ist radial zur bogenförmig verlaufenden Mulde 2 ausgerichtet und erstreckt sich vorne und hinten über den Bereich der Mulde 2 hinaus. Damit ist gewährleistet, dass die gegenüberliegenden Schneidezähne möglichst bei allen vorkommenden Zahn- und Kieferstellungen die Kontaktfläche 5 berühren und dadurch ein direkter Kontakt gegenüberliegender Schneidezähne und damit auch ein direkter Kontakt neben der Zahnschutzschiene liegender, einander gegenüberliegender Backenzähne, verhindert wird. Damit ist es auch möglich, die Zahnschutzschiene wahlweise zum Tragen an den Schneidezähnen des Oberkiefers oder des Unterkiefers einzurichten. Die Kontaktfläche 5 ist dabei auf der Vorderseite an einer über die Vorderwand 9 nach vorne hinaus ragenden Nase 4 und an der Rückseite an einer über die Rückwand 10 nach hinten hinaus ragende Kuppel 3 angeordnet. Die Breite der Kontaktfläche 8 beträgt etwa 6 mm. Wie die Figur zeigt, ragt die Kontaktfläche 5 um ein Mass 6 nach unten über die äussere Wandfläche 8 der Mulde 2 hinaus. Die so gebildete Kante 7 stellt bei Lateralbewegungen des Unterkiefers ein unerwünschtes Hindernis dar, ganz besonders wenn die die Kontaktfläche berührenden Schneidezähne nicht genau gleich lang sind. Selbst wenn die Flächen 5 und 8 keinen Höhenversatz aufweisen, sind bei dieser Zahnschutzschiene 1 im Bereich der Nase 4 und der Kuppel 3 die Kanten 7 immer noch vorhanden und können, je nach Zahn- und Kieferstellung, ein unerwünschtes Hindernis für laterale Kieferbewegungen bilden.

Figur 2 zeigt ein Ausführungsbeispiel der erfindungsgemässen Zahnschutzschiene 12 in einer perspektivischen Ansicht, etwa im gleichen Winkel gesehen wie die Zahnschutzschiene 1 nach Figur 1. Auch diese Zahnschutzschiene 12 hat eine Mulde 13 zur Aufnahme von oberen oder unteren Schneidezähnen. Die Mulde 13 hat eine Bodenfläche 14 und ist durch eine Frontwand 15 und eine Rückwand 16 begrenzt. Links unten in der Figur ist eine Nase 18 zu erkennen, die dadurch gebildet ist, dass die Dicke der Frontwand 15 in einem mittleren Bereich von oben nach unten zunimmt. Die gesamte untere Fläche der Zahnschutzschiene 12 ist als Kontaktfläche 17 für Schneidezähne ausgebildet, die den im Gebrauch in der Mulde 13 aufgenommenen Schneidezähnen gegenüberliegen. Am oberen Rand der Frontwand 15 ist eine Ausnehmung 19 angeordnet, die verhindert, dass beim Tragen der Zahnschutzschiene 12 an den oberen Schneidezähnen das obere Lippenbändchen (Frenulum labii superioris) beziehungsweise beim Tragen der Zahnschutzschiene 12 an den unteren Schneidezähnen das untere Lippenbändchen (Frenulum labii inferioris) durch die freie Kante der Frontwand 15 irritiert und/oder gereizt wird. Am oberen Rand der Rückwand 16 ist eine Ausnehmung 20 angeordnet, die verhindert, dass beim Tragen der Zahnschutzschiene 12 an den oberen Schneidezähnen die Erhebung am vorderen Gaumenende (Papilla incisiva) beziehungsweise beim Tragen der Zahnschutzschiene 12 an den unteren Schneidezähnen das Zungenbändchen (Frenulum linguale) durch die freie Kante der Rückwand 16 irritiert und/oder gereizt wird.

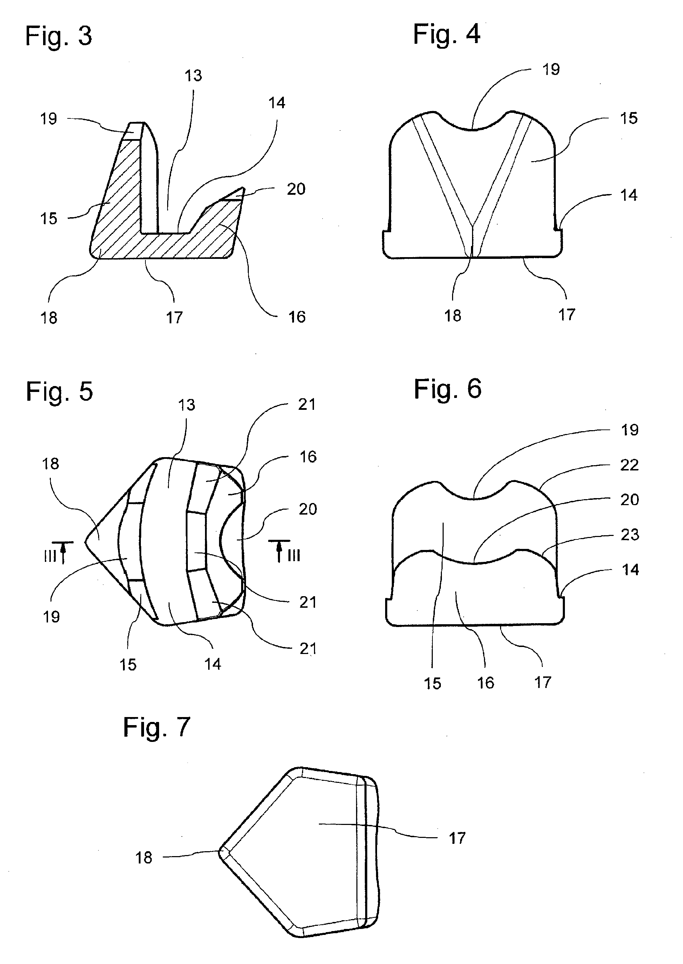

Figur 3 zeigt eine entlang der Linie III - III in Figur 5 geschnittene Ansicht der Zahnschutzschiene 12. Sowohl die Mulde 13 mit ihrer Bodenfläche 14 als auch die vorangehend beschriebenen Ausnehmungen 19 und 20 in der Frontwand 15 beziehungsweise Rückwand 16 sind deutlich zu erkennen. Die Frontwand 15 nimmt in der Schnittebene von oben nach unten in ihrer Dicke zu und bildet auf der Höhe der Kontaktfläche 17 eine Nase 18. Die Bodendicke zwischen der Bodenfläche 14 der Mulde 13 und der Kontaktfläche 17 beträgt im dargestellten Beispiel etwa 1 mm. Es ist vorteilhaft, wenn diese Bodendicke möglichst gering ist, weil dadurch das Risiko sinkt, dass durch das Tragen der Zahnschutzschiene 12 auf Dauer ein offener Biss kreiert wird.

Figur 4 zeigt die Zahnschutzschiene 12 von vorne, wobei die Frontwand 15 mit ihrer oberen Ausnehmung 19 im Vordergrund und beiderseits der Frontwand 15 ein kleiner Abschnitt der Bodenfläche 14 der Mulde 13 sichtbar ist. Der V-förmige Abschnitt der Frontwand 15 ist der Bereich, in dem die Dicke der Frontwand 15 von oben nach unten unter Bildung der Nase 18 zunimmt. In den neben dem V-förmigen Bereich liegenden Bereichen der Frontwand 15 ist diese vom oberen Rand bis in den Bereich der Bodenfläche 14 der Mulde etwa gleich dick.

In Figur 5 ist die Zahnschutzschiene 12 mit Blick von oben dargestellt, so dass die Anordnung und der Verlauf der Mulde 13 besonders deutlich zu sehen sind. Die Bogenlänge der Mulde auf der Bodenfläche 14 am Rand zur Frontwand 15 beträgt im dargestellten Beispiel etwa 18 mm. Deutlich ist in dieser Figur auch zu sehen, dass die Frontwand 15 oben auf nahezu der ganzen Länge der Mulde 13 relativ dünn ist und im mittleren Bereich nach unten zur Nase 18 hin dicker wird, während sie beiderseits der Nase 18 von oben nach unten in etwa gleich dick ist. Die Rückwand 16 hat im dargestellten Beispiel drei der Mulde 13 zugewandte, ebene Teilflächen 21, die relativ zueinander winkelversetzt sind und insgesamt dem Bogenverlauf der Mulde 13 folgen. Die der Mulde 13 zugewandte Fläche der Rückwand kann aber auch einen gebogenen oder geraden Verlauf haben. Die der Mulde 13 abgewandte Fläche der Rückwand 16 hat im dargestellten Beispiel einen geraden Verlauf.

Figur 6 zeigt die Zahnschutzschiene 12 von hinten. Im Vordergrund befindet sich die Rückwand 16, wobei beiderseits davon ein kleiner Abschnitt der Bodenfläche 14 der Mulde 13 sichtbar ist. Hinter der Rückwand 16 ragt die höhere Frontwand 15 hervor, wobei auch die beiden Ausnehmungen 19 und 20 deutlich zu erkennen sind. Sowohl die Rückwand 16 als auch die Frontwand 15 weisen an ihren freien, oberen Rändern beiderseits Abrundungen 22 beziehungsweise 23 auf.

Schliesslich zeigt Figur 7 eine Ansicht von unten, das heisst auf die Kontaktfläche 17 der Zahnschutzschiene 12. Diese Ansicht macht besonders deutlich, dass die Kontaktfläche die gesamte Projektionsfläche der Zahnschutzschiene 12 bedeckt.

Die beschriebene Zahnschutzschiene 12 besteht vorzugsweise aus einem Kunststoff und ist vorteilhaft im Spritzgussverfahren hergestellt.

Zur Anwendung wird eine aushärtbare Masse in die Mulde 13 der Zahnschutzschiene 12 gebracht und dann die Zahnschutzschiene 12 über die Schneidezähne einer Person gestülpt, derart, dass die Schneidezähne in die Mulde 13 zu liegen kommen und die noch plastische Masse sich vorne und hinten genau an die Schneidezähne anpasst. Nach dem Aushärten der Masse wird die Zahnschiene, die nun exakt über die Schneidezähne der betreffenden Person passt, herausgenommen und es wird gegebenenfalls überschüssige Masse, die beispielsweise seitwärts aus der Mulde gedrängt wurde, entfernt. Auch kann die Zahnschutzschiene 12 bei Bedarf noch nachträglich individuell bearbeitet werden, beispielsweise durch Kürzen und/oder Abrunden der Nase 18.

### Bezugszeichenliste

- 1: Zahnschutzschiene
- 2: Mulde
- 3: Kuppel
- 4: Nase
- 5: Kontaktfläche
- 6: Mass
- 7: Kante
- 8: äussere Wandfläche
- 9: Vorderwand
- 10: Rückwand
- 11:
- 12: Zahnschutzschiene
- 13: Mulde
- 14: Bodenfläche der Mulde
- 15: Frontwand
- 16: Rückwand
- 17: Kontaktfläche
- 18: Nase
- 19: Ausnehmung
- 20: Ausnehmung
- 21: Innenfläche Rückwand
- 22: Abrundung
- 23: Abrundung
- 24:
- 25:

## Patentansprüche

1. Vorgefertigte Zahnschutzschiene (12) mit einer durch eine Vorderwand (15) und eine Rückwand (16) begrenzten Mulde (13) zur Aufnahme von Schneidezähnen einer Person, und einer zur Anlage an den in der Mulde (13) aufgenommenen Schneidezähnen gegenüberliegenden Schneidezähnen bestimmte Kontaktfläche (17), wobei die Kontaktfläche (17) in einer Richtung rechtwinklig zum Verlauf der Mulde (13) zumindest in der Längsmitte der Mulde (13) deren Breite auf beiden Seiten überragt, **dadurch gekennzeichnet, dass** sich die Kontaktfläche (17) zumindest im Bereich der Mulde (13) über deren ganze Länge erstreckt.

2. Zahnschutzschiene (12) nach Anspruch 1, **dadurch gekennzeichnet, dass** die Kontaktfläche (17) eine ebene Fläche ist.

3. Zahnschutzschiene (12) nach einem der vorangehenden Ansprüche, **dadurch gekennzeichnet, dass** in der Längsmitte der Mulde (13) die Frontwand (15) von ihrem freien Rand in Richtung auf die Kontaktfläche (17) in ihrer Dicke zunimmt und dass die Dicke der Frontwand (15) im Bereich der Kontaktfläche (17) von der Mitte aus zu beiden Enden der Mulde (13) hin stetig abnimmt.

4. Zahnschutzschiene (12) nach einem der vorangehenden Ansprüche, **dadurch gekennzeichnet, dass** die Rückwand (16) von ihrem freien Rand in Richtung auf die Kontaktfläche (17) in ihrer Dicke zunimmt.

5. Zahnschutzschiene (12) nach einem der vorangehenden Ansprüche, **dadurch gekennzeichnet, dass** die der Mulde abgewandte Seite der Rückwand (16) eine ebene Fläche ist.

6. Zahnschutzschiene (12) nach einem der vorangehenden Ansprüche, **dadurch gekennzeichnet, dass** der freie Rand der Frontwand (15) in deren Mitte eine Ausnehmung (19) aufweist.

7. Zahnschutzschiene (12) nach einem der vorangehenden Ansprüche, **dadurch gekennzeichnet, dass** der freie Rand der Rückwand (16) in deren Mitte eine Ausnehmung (20) aufweist.

8. Zahnschutzschiene (12) nach einem der vorangehenden Ansprüche, **dadurch gekennzeichnet, dass** die Länge der Mulde (13) gemessen an der Bodenfläche (14) der Mulde (13) entlang der Vorderwand zwischen 16 mm und 35 mm·beträgt.

9. Zahnschutzschiene (12) nach einem der vorangehenden Ansprüche, **dadurch gekennzeichnet, dass** die Distanz von der Bodenfläche (14) der Mulde (13) bis zur Kontaktfläche (17) zwischen 0,5 mm und 3 mm beträgt.

## Claims

1. Prefabricated tooth protection guard (12), comprising a trough (13) that is limited by a front wall (15) and a rear wall (16) and intended to receive the incisors of a person, and a contact surface (17) intended to lie against the incisors opposite the incisors received in the trough (13), the contact surface (17) extending, in a direction perpendicularly to the extension of the trough (13), beyond the width of the latter on both sides at least in the longitudinal centre of the trough (13), **characterised in that** at least in the area of the trough (13), the contact surface (17) extends over the entire length thereof.

2. Tooth protection guard (12) according to claim 1, **characterised in that** the contact surface (17) is a plane surface.

3. Tooth protection guard (12) according to one of the preceding claims, **characterised in that** in the longitudinal centre of the trough (13), the front wall (15) increases in thickness from its free edge towards the contact surface (17), and that the thickness of the front wall (15) continuously decreases in the area of the contact surface (17) from the centre to both ends of the trough (13).

4. Tooth protection guard (12) according to one of the preceding claims, **characterised in that** the rear wall (16) increases in thickness from its free edge towards the contact surface (17).

5. Tooth protection guard (12) according to one of the preceding claims, **characterised in that** the side of the rear wall (16) facing away from the trough is a plane surface.

6. Tooth protection guard (12) according to one of the preceding claims, **characterised in that** the free edge of the front wall (15) has a recess (19) in its centre.

7. Tooth protection guard (12) according to one of the preceding claims, **characterised in that** the free edge of the rear wall (16) has a recess (20) in its centre.

8. Tooth protection guard (12) according to one of the preceding claims, **characterised in that** the length of the trough (13), measured on the bottom surface (14) of the trough (13) along the front wall, is comprised between 16 mm and 35 mm.

9. Tooth protection guard (12) according to one of the preceding claims, **characterised in that** the distance between the bottom surface (14) of the trough (13) and the contact surface (17) is comprised between 0.5 mm and 3 mm.

## Revendications

1. Protège-dents (12) préfabriqué, comprenant un creux (13) limité par une paroi avant (15) et une paroi arrière (16) et destiné à recevoir les incisives d'une personne, et une surface de contact (17) destinée à venir se poser sur les incisives opposées aux incisives reçues dans ledit creux (13), la surface de contact (17) dépassant, dans une direction perpendiculaire à l'étendue du creux (13), des deux côtés la largeur de celui-ci du moins au centre longitudinal du creux (13), **caractérisé en ce que** du moins dans la région du creux (13), la surface de contact (17) s'étend sur la longueur entière de celui-ci.

2. Protège-dents (12) selon la revendication 1, **caractérisé en ce que** la surface de contact (17) est une surface plane.

3. Protège-dents (12) selon l'une des revendications précédentes, **caractérisé en ce qu'**au centre longitudinal du creux (13), la paroi avant (15) augmente en épaisseur depuis son bord libre vers la surface de contact (17) et que l'épaisseur de la paroi avant (15) diminue continuellement dans la région de la surface de contact (17) depuis le centre vers les deux extrémités du creux (13).

4. Protège-dents (12) selon l'une des revendications précédentes, **caractérisé en ce que** la paroi arrière (16) augmente en épaisseur depuis son bord libre vers la surface de contact (17).

5. Protège-dents (12) selon l'une des revendications précédentes, **caractérisé en ce que** le côté de la paroi arrière (16) opposé au creux est une surface plane.

6. Protège-dents (12) selon l'une des revendications précédentes, **caractérisé en ce que** le bord libre de la paroi avant (15) présente un évidement (19) dans son centre.

7. Protège-dents (12) selon l'une des revendications précédentes, **caractérisé en ce que** le bord libre de la paroi arrière (16) présente un évidement (20) dans son centre.

8. Protège-dents (12) selon l'une des revendications précédentes, caractérisé en ce la longueur du creux (13), mesurée à la surface inférieure (14) du creux (13) le long de la paroi avant, est comprise entre 16 mm et 35 mm.

9. Protège-dents (12) selon l'une des revendications précédentes, **caractérisé en ce que** la distance entre la surface inférieure (14) du creux (13) et la surface de contact (17) est comprise entre 0,5 mm et 3 mm.
